# EUROPÄISCHE PATENTSCHRIFT

(11) **EP 1 515 441 B1**
(45) Veröffentlichungstag und Bekanntmachung des Hinweises auf die Patenterteilung: **20.01.2010**
(21) Anmeldenummer: 04090309.8
(22) Anmeldetag: 09.08.2004
(51) Int. Cl.: H03K 17/10, A61N 1/362

(54) **Spannungsfester MOS-Schalter**
Voltage resistant MOS switch
Commutateur MOS résistant aux tensions

(30) Priorität: 05.09.2003 DE 10341940; 05.12.2003 DE 10358048
(43) Veröffentlichungstag der Anmeldung: 16.03.2005
(73) Patentinhaber: Biotronik GmbH & Co. KG, 12359 Berlin (DE)
(72) Erfinder: Feese, Ulrich, 14167 Berlin (DE); Kessler, Robert, 10551 Berlin (DE); Wrana, Michael, 12207 Berlin (DE)
(74) Vertreter: Lindner-Vogt, Karin L.

(56) Entgegenhaltungen:
- EP-A- 0 308 536
- US-A- 4 487 457
- US-A- 4 864 126
- US-A- 5 148 064
- US-A1- 2003 016 072
- "INTERRUPTEUR A 5 MOSFET SERIE 2000 V/15 A" ELECTRONIQUE, CEP COMMUNICATION, PARIS, FR, Nr. 3, 1. Dezember 1990 (1990-12-01), Seiten 56-58,60, XP000177309 ISSN: 1157-1152
- DATABASE WPI Section EI, Week 198046 Derwent Publications Ltd., London, GB; Class U21, AN 1980-L0370C XP002297437 -& SU 723 782 A (KHARKOV POLY) 25. März 1980 (1980-03-25)

## Beschreibung

Die Erfindung betrifft einen spannungsfesten Schalter mit einem ersten Schaltkontakt und einem zweiten Schaltkontakt, einem ersten MOS-Schalttransistor mit einem Source-Anschluss, einem Drain-Anschluss und einem Gate-Anschluss, wobei der Source-Anschluss des ersten MOS-Schalttransistors mit dem zweiten Schaltkontakt und der Drain-Anschluss des ersten MOS-Schalttransistors mit dem ersten Schaltkontakt verbunden sind. Der spannungsfeste Schalter ist zum Schalten eines an den ersten oder zweiten Schaltkontakt anzulegenden oder anliegenden Potentials ausgebildet.

Bei MOS-Bauelementen stellt sich aufgrund der fortschreitenden Strukturverkleinerungen und den damit verbundenen geringeren Oxiddicken bei den Gate-Anschlüssen das Problem, dass die Versorgungsspannung für moderne Schaltkreise immer weiter reduziert werden muss. Zu hohe Versorgungsspannungen können durch die Veränderung der elektrischen Eigenschaften zur Schädigung oder sogar zur Zerstörung von MOS-Bauelementen führen. Die Spannungsfestigkeit hinsichtlich der Drain-Source-Kontakte bei sogenannten Hochspannungs-MOS-Transistoren wird im wesentlichen durch niedrig dotierte Drift-Gebiete erreicht. Um die elektrischen Eigenschaften der MOS-Transistoren nicht zu verschlechtern, kann jedoch die Oxiddicke zur Verbesserung der Spannungsfestigkeit nicht beliebig erhöht werden. Um ein Spannungssignal mit einem MOS-Transistor möglichst ohne Beeinflussung durchschalten zu können, muss unter Berücksichtigung der Schwellspannung des MOS-Transistors, beispielsweise im Falle eines NMOS-Transistors, der Betrag des Gate-Potentials größer sein als das Drain- oder Source-Potential. Bei Multiplexern oder Ausgangsschaltern ist es jedoch oftmals nötig Spannungen zu schalten, die größer als die technologisch maximal zulässige Gate-Source-Spannung sind. Das Problem stellt sich auch bei Herzschrittmachern oder Defibrillatoren, bei denen Stimulations- oder Defibrillationssignale geschaltet werden müssen, welche einen großen Dynamikbereich umfassen. Dabei kann die maximal zulässige Gate-Source-Spannung überschritten werden, was zur Schädigung oder Zerstörung des Transistors führen kann.

Aus der DE 37 88 876 T2 ist ein programmierbarer Herzschrittmacher bekannt, welcher durch logische Glieder steuerbare MOS-Schalter aufweist. Das Problem, mit MOS-Schaltern nur Signale mit Signalspannungen innerhalb eines begrenzten Spannungsbereiches schalten zu können wird hier nicht erwähnt und auch nicht gelöst.

Aus US 5148064, US 2003/0016072 und US 4487457 sind weitere Schaltungen bekannt.

Die der Erfindung zugrunde liegende Aufgabe besteht darin, einen Schalter mit MOS-Transistoren anzugeben, welcher eine erhöhte Spannungsfestigkeit aufweist.

Die Aufgabe wird durch einen spannungsfesten Schalter der Eingangs genannten Art gelöst, wobei der spannungsfeste Schalter eine Schalt-Kontrolleinheit mit einem Steuereingang und einem Sicherungsausgang aufweist. Außerdem weist der spannungsfeste Schalter einen Sicherungs-Schalter mit einem Schalteingang auf, der mit dem Sicherungsausgang der Schalt-Kontrolleinheit verbunden ist. Der Sicherungs-Schalter ist angeordnet und ausgebildet, den Gate-Anschluss des ersten MOS-Schalttransistors mit dem Source-Anschluss des ersten MOS-Schalttransistors in Abhängigkeit von einem Sicherungssignal elektrisch zu verbinden, so dass Gate-Anschluss und Source-Anschluss des ersten MOS-Schalttransistors über einen ausreichend kleinen Widerstand miteinander verbunden werden können.

Die Schalt-Kontrolleinheit ist ausgebildet, ein entsprechendes, den Sicherungs-Schalter in seine Gate- und Source-Anschluss des ersten MOS-Schalttransistors verbindenden Schaltzustand bewirkendes Sicherungssignal dann zu erzeugen und am Sicherungsausgang auszugeben.

Erfindungsgemäß weist der spannungsfeste Schalter einen zweiten MOS-Sicherungstransistor auf, welcher mit einem ersten MOS-Sicherungstransistor derart in Reihe geschaltet ist, dass der Source-Anschluss des zweiten MOS-Sicherungstransistors mit dem Source-Anschluss des ersten MOS-Sicherungstransistors - bevorzugt direkt - verbunden ist, so dass der Drain-Anschluss des zweiten MOS-Sicherungstransistors mit dem Source-Anschluss des ersten MOS-Schalttransistors und der Drain-Anschluss des ersten MOS-Sicherungstransistors mit dem Gate-Anschluss des ersten MOS-Schalttransistors und ggf. auch eines zweiten MOS-Schalttransistors- bevorzugt direkt - verbunden ist und der Gate-Anschluss des ersten MOS-Sicherungstransistors - bevorzugt direkt - mit dem Gate-Anschluss des zweiten MOS-Sicherungstransistors verbunden ist. In dieser Anordnung leiten die Sicherungstransistoren gleichzeitig oder sperren gemeinsam. Dadurch wird vorteilhafterweise unabhängig von der Potentialrichtung eine Sperrung der Sicherungstransistoren bewirkt.

Der spannungsfeste Schalter weist einen MOS-Schalttransistor auf, der hier als erster MOS-Schalttransistor bezeichnet wird.

Die Schalt-Kontrolleinheit ist in einer Ausführungsvariante ausgebildet, im Sperrfall des ersten MOS-Schalttransistors den Gate-Anschluss des ersten MOS-Sicherungstransistors derart mit einem Durchschaltpotential zu beaufschlagen, dass der erste MOS-Sicherungstransistor durchsteuert und im Falle der Durchsteuerung des ersten MOS-Schalttransistors den Gate-Anschluss des ersten MOS-Sicherungstransistors derart mit einem Sperrpotential zu beaufschlagen, dass der erste MOS-Sicherungstransistor sperrt.

In einer anderen Variante ist die Schalt-Kontrolleinheit ausgebildet, bei Überschreiten einer vorbestimmten Potentialdifferenz zwischen dem Source-Anschluss und dem Gate-Anschluss des ersten MOS-Schalttransistors oder zwischen dem ersten Schaltkontakt und dem zweiten Schaltkontakt, den Gate-Anschluss des ersten MOS-Sicherungstransistors derart mit einem Durchschaltpotential zu beaufschlagen, dass der erste MOS-Sicherungstransistor - vorzugsweise vollständig - durchsteuert und bei Unterschreiten der vorbestimmten Potentialdifferenz zwischen dem ersten Schaltkontakt und dem zweiten Schaltkontakt des spannungsfesten Schalters oder im Falle der Durchsteuerung des Schalttransistors den Gate-Anschluss des ersten MOS-Sicherungstransistors derart mit einem Sperrpotential zu beaufschlagen, dass der erste MOS-Sicherungstransistor - vorzugsweise vollständig - sperrt.

Diese Schaltungsanordnung für einen spannungsfesten Schalter bewirkt, dass im Sperrfall der Gate-Anschluss und der Source-Anschluss des Schalttransistors kurzgeschlossen sind. Dadurch wird vorteilhaft vermieden, dass die maximal zulässige Spannung überschritten wird, welche im Sperrfall zwischen dem Source-Kontakt und dem Gate-Kontakt des Schalttransistors abfallen darf. Es ist somit ein erfindungswesentliches Merkmal, dass der spannungsfeste Schalter bei größeren Potentialdifferenzen zwischen den Schaltkontakten betrieben werden kann als es die Spannungsfestigkeit der einzelnen Schalttransistoren zulässt, ohne dass die Schalttransistoren einen Schaden erleiden.

Unhabhängig davon, ob der Spannungsfeste Schalter einen Sicherungs-Schalter der zuvor beschriebenen Art aufweist, und unabhängig davon ob die Schaltkontrolleinheit einen entsprechenden Sicherungs-Ausgang besitzt, kann die Schalt-Kontrolleinheit einen Steuerausgang aufweisen, welcher mit dem Gate-Anschluss des MOS-Schalttransistors verbunden ist. Die Schalt-Kontrolleinheit ist ausgebildet, den Gate-Anschluss des ersten MOS-Schalttransistors mit einem derartigen Steuerpotential zu beaufschlagen, welches ein Durchsteuern oder ein Sperren, insbesondere ein vollständiges Durchsteuern oder ein vollständiges Sperren des ersten MOS-Schalttransistors zwischen dem Source-Anschluss und dem Drain-Anschluss bewirkt.

Die Schalt-Kontrolleinheit kann hierzu eingangsseitig mit dem ersten Schaltkontakt und dem zweiten Schaltkontakt mindestens mittelbar verbunden sein. Dadurch kann die Schalt-Kontrolleinheit ein an einem Schaltkontakt anliegendes Potential erfassen.

Die Schalt-Kontrolleinheit ist bevorzugt ausgebildet, das Steuerpotential aus einer Anzahl vorbestimmter Steuerpotentiale in Abhängigkeit von einem vorbestimmten Schaltpotential oder einem an dem ersten Schaltkontakt anzulegenden oder anliegenden ersten, oder einem an dem zweiten Schaltkontakt anzulegenden oder anliegenden zweiten Potential oder beiden in vorgegebener Weise auszuwählen, und zwar vorzugsweise aus einer Anzahl vorbestimmter Steuerpotentiale gemäß einer vorbestimmten Zuordnungsvorschrift.

Beispielhafte Ausführungsformen für eine schaltungstechnische Realisierung einer Zuordnungsvorschrift können Netzwerke mit einem Eingang und einem Ausgang, umfassend passive und/oder aktive Bauelemente sein, welche eine die Zuordnungsvorschrift repräsentierende Übertragungsfunktion zwischen einem Ausgang und einem Eingang aufweisen.

In einer anderen Ausführungsform sind die vorbestimmten an dem ersten und/oder dem zweiten Schaltkontakt anliegenden oder anzulegenden Potentiale und die zugehörigen Schaltpotentiale in einer Look-up-Tabelle oder in Form eines Steuerprogrammes verwirklicht.

Ein Beispiel für eine bevorzugte Zuordnungsvorschrift für ein in Abhängigkeit von einem an einem Schaltkontakt anzulegenden Potential auszuwählendes Gate-Potential ist in der folgenden Tabelle dargestellt:

| Ux[V] | UG[V] |
|---|---|
| 0 | U |
| -U | U |
| -U | 0 |
| -2*U | 0 |
| -2*U | -U |
| -3*U | -U |

In der vorstehenden Tabelle sind die an einem Schaltkontakt anzulegenden oder anliegenden Potentiale als Ux und die jeweils entsprechenden Gate-Potentiale zum Schalten als UG bezeichnet, jeweils bezogen auf einen gemeinsamen Bezugsknoten. Die in der Tabelle angegebene Spannung U kann eine in einem Gerät vorgegebene Spannung sein. Denkbar sind auch positive an einem Schaltkontakt anzulegende oder anliegende Potentiale.

Alternativ dazu kann die Schalt-Kontrolleinheit ausgebildet sein, das Steuerpotential aus einer Anzahl vorbestimmter Steuerpotentiale derart auszuwählen, dass das Steuerpotential zu einem an dem ersten Schaltkontakt anliegenden oder anzulegenden ersten Potential und einem an dem zweiten Schaltkontakt anliegenden oder anzulegenden zweiten Potential in einem vorbestimmten Verhältnis steht.

Die vorig beschriebenen Ausführungsvarianten bewirken vorteilhaft, dass der MOS-Schalttransistor im Triodenbereich betreibbar ist und keine Signalverzerrungen auftreten, welche sich beim Betreiben des MOS-Schalttransistors oberhalb des Pinch-Off-Betriebspunktes einstellen würden.

In einer bevorzugten Ausführungsvariante weist der spannungsfeste Schalter einen zweiten MOS-Schalttransistor auf. Der zweite MOS-Schalttransistor ist mit dem ersten MOS-Schalttransistor derart in Reihe geschaltet, dass der Source-Anschluss des zweiten MOS-Schalttransistors mit dem Source-Anschluss des ersten MOS-Schalttransistors - bevorzugt direkt - verbunden ist und der Drain-Anschluss des ersten MOS-Schalttransistors - bevorzugt direkt - mit dem ersten Schaltkontakt und der Drain-Anschluss des zweiten MOS-Schalttransistors - bevorzugt direkt - mit dem zweiten Schaltkontakt verbunden ist.

In einer weiter bevorzugten Ausführungsform ist der Gate-Anschluss des ersten Schalttransistors mit dem Gate-Anschluss des zweiten MOS-Schalttransistors - bevorzugt direkt - verbunden.

Alternativ dazu kann der Gate-Anschluss des zweiten Schalttransistors auch mit der Schalt-Kontrolleinheit verbunden sein, welche in diesem Fall ausgebildet ist, in Abhängigkeit von einem an dem ersten Schaltkontakt anzulegenden oder anliegenden Potential und/oder einem an dem zweiten Schaltkontakt anzulegenden oder anliegenden Potential die Gate-Anschlüsse der Schalttransistoren jeweils unabhängig voneinander mit einem für den jeweiligen Schalttransistor vorbestimmten Steuerpotential zu beaufschlagen.

In einer weiter bevorzugten Ausführung weist die Schalt-Kontrolleinheit einen Mittenpotentialanschluss auf, welcher mit den Source-Anschlüssen der Schalttransistoren verbunden ist, wobei die Schalt-Kontrolleinheit ausgebildet ist, beim Sperren der Schalttransistoren den Mittenpotentialanschluss mit einem Mittenpotential derart zu beaufschlagen, dass jeweils eine vorbestimmte Potentialdifferenz zwischen dem Mittenpotentialanschluss und den Gate-Anschlüssen der Schalttransistoren nicht überschritten wird. Die vorbestimmte Potentialdifferenz ist vorzugsweise kleiner als die maximal zulässige Gate-Source-Spannung der Schalttransistoren. Eine besonders bevorzugte Potentialdifferenz ist 0 Volt.

Im Falle einer Anordnung mit zwei Schalttransistoren fällt eine an dem ersten Schaltkontakt und an dem zweiten Schaltkontakt anliegende Spannung über dem ersten Schalttransistor und dem zweiten Schalttransistor ab, so dass in dieser Anordnung die Anforderungen an einen Schalttransistor hinsichtlich der Spannungsfestigkeit geringer sind. In dieser Anordnung leiten die Schalttransistoren gleichzeitig oder sperren gemeinsam.

Die Gate-Anschlüsse der beiden MOS-Sicherungstransistoren können in alternativen Ausführungsvarianten, bei denen die beiden Gate-Anschlüsse nicht direkt miteinander verbunden sind, auch mit unterschiedlichen Potentialen angesteuert werden.

In einer bevorzugten Ausführungsform ist bei jedem MOS-Schalttransistor ein Bulk-Anschluss vorhanden, welcher vorzugsweise mit dem Source-Anschluss des jeweiligen MOS-Schalttransistors verbunden ist. Dadurch kann vorteilhaft ein größerer Strom auf der Source-Drain-Strecke eines jeden Schalttransistors fließen.

In einer weiter bevorzugten Ausführungsform ist bei jedem MOS-Sicherungstransistor ein Bulk-Anschluss vorhanden, welcher vorzugsweise mit dem Source-Anschluss des jeweiligen MOS-Sicherungstransistors verbunden ist. Dadurch kann im Falle einer niederohmigen Verbindung des Gate-Anschlusses und des Source-Anschlusses des Schalttransistors ein erhöhter Kurzschlussstrom durch die Sicherungstransistoren fließen.

In einer bevorzugten Ausführungsvariante sind die MOS-Schalttransistoren und die MOS-Sicherungstransistoren als unsymmetrische MOS-Transistoren ausgebildet. Der spannungsfeste Schalter ist somit bezüglich der Schaltkontakte symmetrisch aufgebaut und arbeitet unabhängig von der Polarität der Spannung, die über den Schaltkontakten anliegt.

In einer alternativen Ausführungsform sind die MOS-Schalttransistoren und die MOS-Sicherungstransistoren als Insulated Gate Bipolar-Transistoren ausgebildet. In dieser Ausführungsform kann der spannungsfeste Schalter vorteilhaft hohe Spannungen und große Ströme schalten.

In einer besonders bevorzugten Ausführungsform ist der spannungsfeste Schalter monolithisch integriert. In dieser Ausführungsform ist der spannungsfeste Schalter vorteilhaft in einem Baustein integriert.

Bevorzugt sind die MOS-Transistoren des spannungsfesten Schalters als NMOS-Transistoren, weiter bevorzugt als selbstsperrende NMOS-Transistoren ausgebildet. Die MOS-Transistoren können auch als PMOS-Transistoren, weiter bevorzugt als selbstsperrende PMOS-Transistoren ausgebildet sein. Denkbar ist auch eine Ausführungsform, bei welcher die Sicherungstransistoren als selbstleitende MOS-Transistoren ausgebildet sind.

Die Erfindung betrifft auch einen Herzschrittmacher oder einen Defibrillator mit dem erfindungsgemäßen spannungsfesten Schalter. Bei einem Herzschrittmacher oder einem Defibrillator, bei welchem die Spannung und die Polarität eines zu schaltenden Stimulationsimpulses veränderbar ist, lässt sich ein spannungsfester Schalter gemäß einer der vorgenannten Ausführungsformen besonders vorteilhaft einsetzen.

Eine Stimulationseinheit zur Stimulation eines Herzens kann vorteilhaft einen erfindungsgemäßen spannungsfesten Schalter und einen Kondensator zur Speicherung elektrischer Ladung für ein Stimulationssignal aufweisen. Der spannungsfeste Schalter ist hierbei derart mit dem Kondensator wirkverbunden, dass eine Entladung des Kondensators über den spannungsfesten Schalter erfolgen kann. Die Stimulationseinheit kann Bestandteil einer Vorrichtung zur Stimulation eines Herzens, beispielsweise eines Herzschrittmachers oder eines Defibrillators sein.

Die Stimulationseinheit enthält vorteilhaft eine Steuereinheit, welche mit dem spannungsfesten Schalter verbunden und ausgebildet ist, Stimulationssignale zur Stimulation eines Herzens mit dem spannungsfesten Schalter zu schalten. Die Steuereinheit kann hierbei ausgebildet sein, ein Stimulationssignal über den spannungsfesten Schalter auf eine Stimulationselektrode zu schalten.

Die Erfindung soll nun anhand von Figuren näher beschrieben werden.

Figur 1 zeigt schematisch eine Schaltungsanordnung eines spannungsfesten Schalters mit MOS-Transistoren.

Figur 2 zeigt eine schematische Darstellung eines Ausführungsbeispiels einer Schalt-Kontrolleinheit mit zwei Spannungsgeneratoren für einen spannungsfesten Schalter.

Figur 3 zeigt - schematisch dargestellt - ein Ausführungsbeispiel eines Spannungsgenerators für eine Schalt-Kontrolleinheit.

Figur 4 zeigt schematisch eine Stimulationseinheit mit einem spannungsfesten Schalter zur Stimulation eines Herzens.

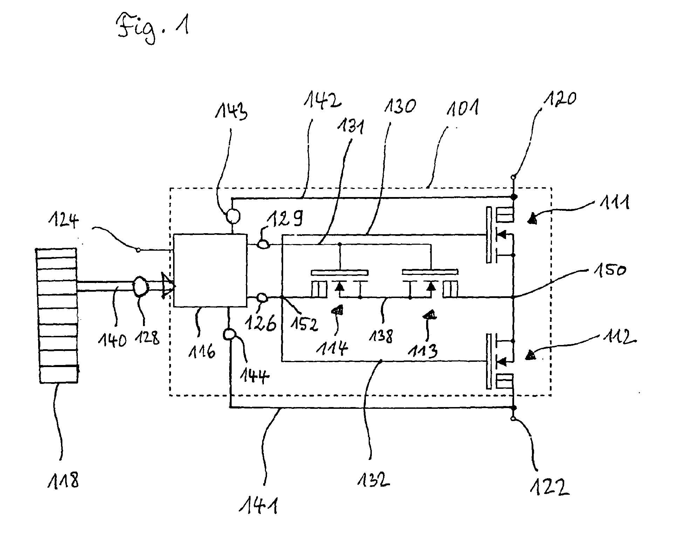

In Figur 1 ist eine Schaltungsanordnung für einen spannungsfesten MOS-Schalter 101 schematisch dargestellt.

Der spannungsfeste Schalter 101 umfasst einen ersten Schaltkontakt 120, einen zweiten Schaltkontakt 122, einen ersten MOS-Schalttransistor 111 und einen zweiten MOS-Schalttransistor 112, deren Source-Anschlüsse über einen Knoten 150 miteinander verbunden sind. Der Drain-Anschluss des ersten Schalttransistors 111 ist mit dem ersten Schaltkontakt 120 verbunden und der Drain-Anschluss des zweiten Schalttransistors 112 ist mit dem zweiten Schaltkontakt 122 verbunden. Die MOS-Schalttransistoren 111 und 112 sind im bevorzugten Ausführungsbeispiel als unsymmetrische MOS-Transistoren ausgebildet, können aber für bestimmte Anwendungen auch symmetrisch ausgebildet sein. Dazu ist die Dotierung der Source-Gebiete im Vergleich zu den Drain-Gebieten verschieden ausgeführt. Bei jedem MOS-Schalttransistor ist der Source-Anschluss mit dem Bulk-Anschluss verbunden. In dieser Anordnung sind die Schalttransistoren bezüglich der Schaltkontakte 120 und 122 symmetrisch angeordnet, so dass der Schalter unabhängig von der Polarität eines zu schaltenden Signales arbeiten kann.

Der spannungsfeste Schalter 101 weist eine Schalt-Kontrolleinheit 116 mit einem Steuereingang 124, einem Steuerausgang 126 und einem Sicherungsausgang 129 auf. Der Steuerausgang 126 ist über einen Knoten 152 und Verbindungsleitungen 130 und 132 mit dem Gate-Anschluss des ersten Schalttransistors 111 und mit dem Gate-Anschluss des zweiten Schalttransistors 112 verbunden. Der spannungsfeste Schalter 101 weist auch einen ersten MOS-Sicherungstransistor 114 und einen zweiten MOS-Sicherungstransistor 113 auf, deren Source-Anschlüsse über eine Verbindungsleitung 138 miteinander verbunden sind und deren Gate-Anschlüsse über eine Verbindungsleitung 131 mit dem Sicherungsausgang 129 der Schalt-Kontrolleinheit 116 verbunden sind. Der Drain-Anschluss des ersten MOS-Sicherungstransistors 114 ist mit dem Knoten 152 und der Drain-Anschluss des zweiten MOS-Sicherungstransistors mit dem Knoten 150 verbunden. Die MOS-Sicherungstransistoren 113 und 114 sind als unsymmetrische MOS-Transistoren ausgebildet und der Source-Anschluss eines jeden MOS-Sicherungstransistors ist mit dem jeweiligen Bulk-Anschluss verbunden. Die Schalt-Kontrolleinheit 116 weist einen Potentialeingang 128 auf, welcher über einen Datenbus 140 mit einem Speicherfeld 118 verbunden ist. Das Speicherfeld 118 enthält diskrete Potentialniveaus, auf welche die Schalt-Kontrolleinheit 116 über den Datenbus 140 wahlfrei zugreifen kann. Die Schalt-Kontrolleinheit 116 weist einen ersten Potentialeingang 143, welcher über eine erste Kontakt-Potentialleitung 142 mit dem ersten Schaltkontakt 120 verbunden ist und einen zweiten Potentialeingang 144 auf, welcher über eine zweite Kontakt-Potentialleitung 141 mit dem zweiten Schaltkontakt 122 verbunden ist. Die Schalt-Kontrolleinheit ist ausgebildet, eventuell in Abhängigkeit von vorbestimmten Potentialen an dem ersten Schaltkontakt 120 und/oder dem zweiten Schaltkontakt 122 ein Durchschaltpotential oder ein Sperrpotential für die Sicherungstransistoren zu erzeugen und den Sicherungsausgang 129 mit diesem Durchschaltpotential oder dem Sperrpotential zu beaufschlagen, so dass bei gesperrten Schalttransistoren das Durchschaltpotential der Sicherungstransistoren so eingestellt wird, dass die Source- und Gate-Anschlüsse der Schalttransistoren 111 und 112 über die MOS-Sicherungstransistoren 113 und 114 niederohmig verbunden sind.

Dadurch sind die Schalttransistoren 111, 112 im Falle deren Sperrung geschützt, wenn der Sicherungsausgang 129 mit einem Durchschaltpotential beaufschlagt ist. In dem in Figur 1 dargestellten Schaltbild sind die MOS-Transistoren als selbstsperrende NMOS-Transistoren ausgebildet.

Alternativ zu der Ausführung als selbstsperrende MOS-Transistoren können die Sicherungstransistoren 113 und 114 auch als selbstleitende MOS-Transistoren ausgebildet sein. In diesem Fall ist die Schalt-Kontrolleinheit 116 ausgebildet, den Sicherungsausgang 129 mit einem Sperrpotential zu beaufschlagen, wenn die Source- und Gate-Anschlüsse der Schalttransistoren 111 und 112 nicht kurzgeschlossen sein sollen.

Die Funktionsweise des spannungsfesten Schalters soll nun im Folgenden erläutert werden:

Wenn am Steuereingang 124 ein Steuersignal zum Durchschalten des spannungsfesten MOS-Schalters 101 anliegt, erzeugt die Schalt-Kontrolleinheit ein Durchschaltpotential und beaufschlagt den Steuerausgang 126 und die angeschlossenen Gate-Anschlüsse der MOS-Schalttransistoren 111 und 112 mit diesem Durchschaltpotential. Die Schalt-Kontrolleinheit ist ausgebildet, das Steuerpotential aus einer Anzahl vorbestimmter Steuerpotentiale in Abhängigkeit von einem an dem ersten Schaltkontakt anzulegenden ersten oder einem an dem zweiten Schaltkontakt anzulegenden zweiten Potential oder beiden gemäß einer Zuordnungsvorschrift auszuwählen, so dass die Arbeitspunkte der MOS-Schalttransistoren im Triodenbereich unterhalb des Pinch-Off-Betriebspunktes liegen. Die vorbestimmten Steuerpotentiale sind in dem Speicherfeld 118 abgelegt und stehen als diskrete Potentialwerte über den Datenbus 140 in digitaler Form zur Verfügung.

Ein Beispiel für die in dem Speicherfeld abgelegten Potentiale ist in der folgenden Tabelle dargestellt, durch deren jeweils entsprechende Werte eine Zuordnungsvorschrift repräsentiert wird:

| Ux | UG |
|---|---|
| | |
| 0 V | 2,8 V |
| - 2,8 V | 2,8 V |
| - 2,8 V | 0 V |
| - 5,6 V | 0 V |
| - 5,6 V | - 2,8 V |
| - 8,4 V | - 2,8 V |

In der vorstehenden Tabelle sind die an einem Schaltkontakt anzulegenden oder anliegenden Potentiale als Ux und die jeweils entsprechenden Gate-Potentiale zum Schalten als UG bezeichnet, jeweils bezogen auf einen gemeinsamen Bezugsknoten.

Alternativ dazu ist auch eine analoge Variante denkbar, wobei das Speicherfeld analoge Signale enthält und der Datenbus 140 für jedes Steuerpotential eine Verbindungsleitung aufweist. Wenn am Steuereingang 124 ein Steuersignal zum Sperren des spannungsfesten MOS-Schalters 101 anliegt, erzeugt die Schalt-Kontrolleinheit ein Sperrpotential und beaufschlagt den Steuerausgang und die angeschlossenen Gate-Anschlüsse der MOS-Schalttransistoren 111 und 112 mit diesem Sperrpotential. Überschreitet im Sperrfall der Schalttransistoren 111, 112 die Potentialdifferenz zwischen den Schaltkontakten 120 und 122 einen vorbestimmten Spannungswert, so erzeugt die Schalt-Kontrolleinheit 116 ein Durchschaltpotential und beaufschlagt den Sicherungsausgang 129 und somit die mit diesem über die Verbindungsleitung 136 verbundenen Gate-Anschlüsse der Sicherungstransistoren 113 und 114 mit diesem Durchschaltpotential, so dass die Sicherungstransistoren 113 und 114 vollständig durchgesteuert sind. Dabei kann eine vorbestimmte Potentialdifferenz zwischen den Schaltkontakten 120 und 122 im Sperrfall einer Potentialdifferenz zwischen dem Source-Anschluss und dem Gate-Anschluss eines Schalttransistors entsprechen.

Die Schalt-Kontrolleinheit 116 kann auch ausgebildet sein, im Sperrfall der Schalttransistoren 111, 112 zur Vermeidung einer Überschreitung einer vorbestimmten Potentialdifferenz zwischen dem Source-Anschluss und dem Gate-Anschluss eines Schalttransistors am Sicherungsausgang ein Durchschaltpotential zu erzeugen.

Dadurch sind die Source- und Gate-Anschlüsse der Schalttransistoren 111 und 112 niederohmig miteinander verbunden, so dass eine im Sperrfall des spannungsfesten Schalters über diesen Anschlüssen abfallende Spannung kurzgeschlossen wird und die MOS-Schalttransistoren 111 und 112 nicht beschädigt werden können.

In einer alternativen, nicht dargestellten Ausführung entfallen die Kontakt-Potentialleitungen 141 und 142. Den Potentialeingängen 143 und 144 kann in dieser Ausführungsform jeweils ein einem tatsächlichen Potential an den jeweiligen Schaltkontakten entsprechendes Potentialsignal zugeführt werden, sodass eine galvanische Trennung zwischen den Schaltkontakten und den Potentialeingängen 143 und 144 verwirklicht ist.

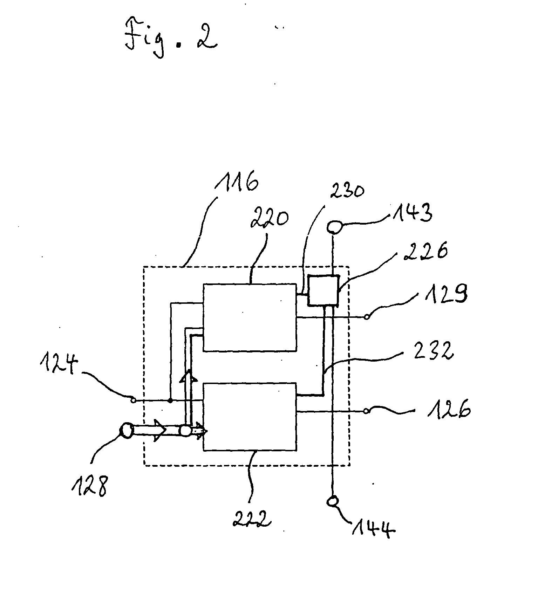

In Figur 2 ist eine detaillierte Ausführungsform der Schalt-Kontrolleinheit schematisch dargestellt.

Die Schalt-Kontrolleinheit enthält, wie in Figur 2 gezeigt, zwei Kontrolleinheiten 220 und 222 sowie eine Steuereinheit 226. In der Steuereinheit 226 ist die für eine Anwendung des spannungsfesten Schalters 101 relevante Zuordnungsvorschrift zwischen anzulegender Gate-Spannung im Bezug auf eine durchzuschaltende Drain-Source-Spannung, beispielsweise schaltungstechnisch oder in einer Look-up-Tabelle, festgelegt.

Die Kontrolleinheit 222 ist ausgebildet, aus einem an dem Steuereingang 124 anliegenden Steuersignal und den von dem Speicherfeld 118 zur Verfügung gestellten diskreten Potentialniveaus ein entsprechendes diskretes Gatesignal für die Transfertransistoren 111 und 112 in Figur 1 gemäß der in der Steuereinheit 226 festgelegten Zuordnungsvorschrift zu erzeugen. Dazu ist die Steuereinheit 226 ausgebildet, ein Zuordnungssignal in Abhängigkeit von an den Schaltkontakten 120 und 122 herrschenden Potentialen gemäß der Zuordnungsvorschrift zu erzeugen und dieses Zuordnungssignal über einen Ausgang und eine Verbindungsleitung 232 an die Kontrolleinheit 222 zu senden. Die Steuereinheit 226 besitzt dazu einen ersten Potentialeingang 143, welcher mit dem ersten Schaltkontakt verbunden ist und einen zweiten Potentialeingang 144, welcher mit dem zweiten Schaltkontakt 122 verbunden ist.

Die Kontrolleinheit 220 ist ausgebildet, je nach Arbeitspunkt der Schalttransistoren 111 und 112 ein Durchschaltpotential oder ein Sperrpotential zu erzeugen und im Sperrfall durch entsprechende Ansteuerung der Sicherungstransistoren 113 und 114 eine niederohmige Verbindung zwischen Gate- und Source-Anschluss der Schalttransistoren 111 und 112 herzustellen. Die Steuereinheit 226 ist ausgebildet, ein das Durchschaltpotential oder das Sperrpotential repräsentierendes Sicherungssignal gemäß einer Zuordnungsvorschrift in Abhängigkeit von den Potentialen an den Potentialeingängen 143 und 144 zu erzeugen und das Sicherungssignal über einen Ausgang und eine Verbindungsleitung 230 an die Kontrolleinheit 220 zu senden. Die Kontrolleinheit 220 ist ausgebildet, aus einem an dem Steuereingang 124 anliegenden Steuersignal und den von dem Speicherfeld 118 zur Verfügung gestellten diskreten Potentialniveaus ein entsprechendes diskretes Gatesignal für die Sicherungstransistoren 113 und 114 in Figur 1 gemäß dem Sicherungssignal, und somit auch gemäß der in der Steuereinheit 226 festgelegten Zuordnungsvorschrift zu erzeugen und den Sicherungsausgang 129 mit einem diskreten Durchschaltpotential oder einem diskreten Sperrpotential zu beaufschlagen.

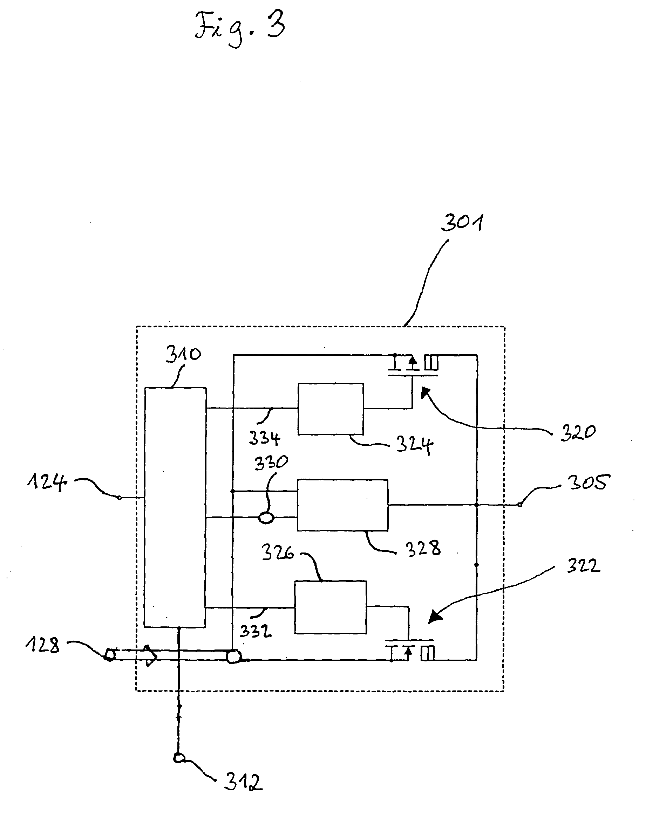

Eine mögliche Kontrollschaltung 301 für die Kontrolleinheiten 220 und 222 ist in Figur 3 dargestellt.

Die Kontrollschaltung 301 enthält eine Auswahleinheit 310 mit einem Steuereingang 124 und einem Zuordnungseingang 312 für das Sicherungssignal oder das Zuordnungssignal, wobei der Zuordnungseingang 312 mit der Steuereinheit 226 verbunden ist. Die Kontrollschaltung enthält für jedes in dem Speicherfeld abgelegte Potentialniveau einen Levelshifter und einen mit diesem verbundenen Transfertransistor zur Erzeugung des diesem Potentialniveau entsprechenden Potentials. Ein Transfertransistor und ein mit diesem verbundener Levelshifter bilden eine Transfer-Einheit.

Die von dem Speicherfeld 118 zur Verfügung gestellten diskreten Potentialniveaus - in diesem Ausführungsbeispiel in analoger Form - werden je nach durchzuschaltender Drain-Source-Spannung über Transfertransistoren 320 und 322 auf den Knoten 305 geschaltet, welcher im Falle der Kontrolleinheit 220 mit dem Sicherungsausgang 129 oder im Falle der Kontrolleinheit 222 mit dem Steuerausgang 126 verbunden ist. Die Transfertransistoren 320 und 322 werden ihrerseits über die passenden digitalen Levelshifter 324 und 326 angesteuert. Die weiteren Potentialniveaus werden über entsprechende, in diesem Ausführungsbeispiel nicht dargestellte Transfer-Einheiten auf den Knoten 305 geschaltet. Die Levelshifter 324 und 326 weisen jeweils einen Ansteuereingang 332 oder 334 auf, welcher mit der Auswahleinheit 310 verbunden ist. Je nach dem, welches Potentialniveau über das an dem Zuordnungseingang 312 anliegende Zuordnungssignal oder Sicherungssignal angesteuert wird, steuert die Auswahleinheit 310 die mit dieser verbundene, dem jeweiligen Potentialniveau entsprechende Transfer-Einheit an. Die Auswahleinheit 310 sorgt somit dafür, dass lediglich eines der in dem Speicherfeld 118 abgelegten Potentialniveaus, welches dem Zuordnungssignal oder dem Sicherungssignal entspricht, über die Transfer-Einheiten in ein dementsprechendes Potential umgesetzt wird und von der Transfer-Einheit auf den Knoten 305 geschaltet wird. Dargestellt ist auch eine Transfer-Einheit 328, welche wie die bereits beschriebenen Transfer-Einheiten einen Levelshifter und einen mit diesem verbundenen Transfertransistor enthalten kann.

Denkbar ist unabhängig von der bisher beschriebenen analogen Variante auch eine Ausführungvariante mit nur einer D/A-Transfer-Einheit anstelle der Transfer-Einheit 328, welche als D/A-Potentialwandler ausgebildet ist. In dieser Ausführungsvariante entfallen die dargestellten Transfer-Einheiten, welche die Levelshifter 324 und 326 und die Transfertransistoren 320 und 322 umfassen. Die Transfer-Einheit 328 ist ausgebildet, über den mit der Auswahleinheit 310 verbundenen Ansteuereingang 330 ein von der Auswahleinheit 310 - entsprechend dem Zuordnungs- oder Sicherungssignal unter Verwendung eines diskreten Potentialniveaus gebildetes - digitales Potentialniveau zu empfangen und ein dementsprechendes analoges Potential zu bilden und den Knoten 305 mit diesem analogen Potential zu beaufschlagen. Die Auswahleinheit 310 ist in dieser Ausführungsvariante ausgebildet, einen an dem Zuordnungseingang 312 anliegenden kontinuierlichen Potentialwert einem diskreten Potentialniveau zuzuordnen und ein dementsprechendes Ausgangssignal zu erzeugen. Der kontinuierliche Potentialwert wird durch das Zuordnungssignal oder das Sicherungssignal repräsentiert.

Die in den Figuren 1, 2 und 3 dargestellten Komponenten können auf einem integrierten Schaltkreis monolithisch ausgeführt sein.

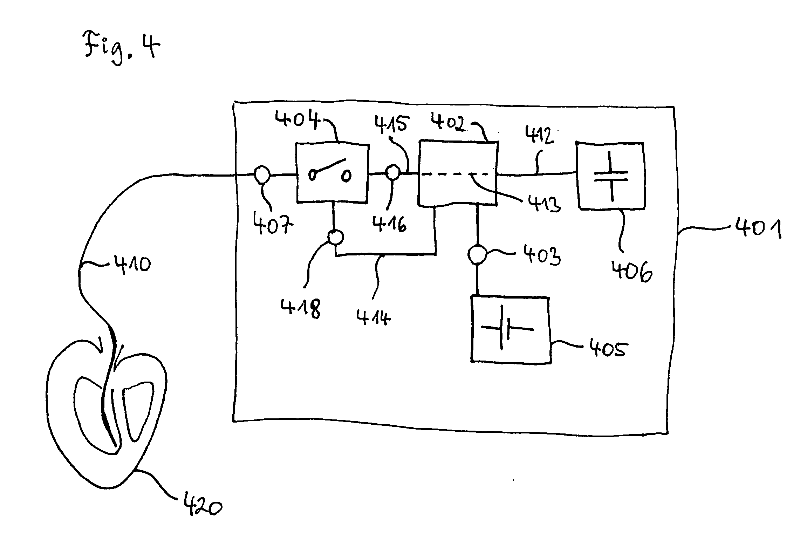

Figur 4 zeigt ein Ausführungsbeispiel für eine Stimulationseinheit 401 zur Stimulation eines Herzens 420. Die Stimulationseinheit 401 enthält eine Steuereinheit 402, welche über eine Verbindungsleitung 412 mit einem Kondensator 406 zur Speicherung von Stimulationsenergie in Form elektrischer Ladung verbunden ist. Die Steuereinheit ist über eine Steuerleitung 414 mit einem Steuereingang 418 und über eine Stimulationsimpulsleitung 415 mit einem ersten Schaltkontakt 416 eines spannungsfesten Schalters 404 verbunden. Ein zweiter Schaltkontakt 407 des spannungsfesten Schalters bildet einen Stimulationsausgang, an welchen eine Stimulationselektrode angeschlossen werden kann. Die Steuereinheit 402 weist einen Stromversorgungseingang 403 zum Anschluss einer Batterie 405 auf. Die Steuereinheit 402 ist ausgebildet, den Lade- und Entladevorgang des Kondensators 406 zur Bereitstellung von Stimulationsenergie zu steuern.

In einer einfachen Ausführungsform ist die Steuereinheit 402 ausgebildet, den Entladevorgang des Kondensators 406 über den spannungsfesten Schalter 404 zu steuern. Dazu kann die Steuereinheit 402 ein Steuersignal erzeugen und dieses über die Steuerleitung 414 auf den Steuereingang 418 des spannungsfesten Schalters 404 schalten. In dieser Ausführung ist die Verbindungsleitung 412 über eine - gestrichelt gezeichnete - Brückenleitung 413 mit der Stimulationsimpulsleitung 415 verbunden.

In einer anderen Ausführungsform kann eine impulsformende Steuereinheit anstelle der Steuereinheit 402 mit der Stimulationsenergie aus dem Kondensator 406 einen Defibrillations- oder Stimulationsimpuls erzeugen und diesen über den spannungsfesten Schalter 404 auf einen Stimulationsausgang 407 und somit auf eine an diesem angeschlossene Stimulationselektrode 410 schalten. Der Stimulationsimpuls ist in dieser Ausführungsform verschieden von einer R-C-Entladungsfunktion und kann beispielsweise patientenspezifisch angepasst werden. In dieser Ausführungsform entfällt die Brückenleitung 413.

## Patentansprüche

1. Spannungsfester Schalter (101) mit
einem ersten Schaltkontakt (120) und einem zweiten Schaltkontakt (122),
einem ersten MOS-Schalttransistor (111) mit einem Source-Anschluss, einem Drain-Anschluss und einem Gate-Anschluss, wobei der Source-Anschluss des ersten MOS-Schalttransistors (111) mit dem zweiten Schaltkontakt (122) und der Drain-Anschluss des ersten MOS-Schalttransistors (111) mit dem ersten Schaltkontakt (120) verbunden sind, wobei der spannungsfeste Schalter zum Schalten eines an den ersten oder zweiten Schaltkontakt anzulegenden oder anliegenden Potentials ausgebildet ist, wobei
der spannungsfeste Schalter (101) eine Schalt-Kontrolleinheit (116) mit einem Steuereingang (124) und einem Sicherungsausgang (129) aufweist, welcher mit einem Schalteingang eines Sicherungs-Schalters (114) verbunden ist, wobei dieser Sicherungs-Schalter (114) derart angeordnet und ausgebildet ist, dass er den Gate-Anschluss des ersten MOS-Schalttransistors (111) mit dem Source-Anschluss des ersten MOS-Schalttransistors (111) in Abhängigkeit von einem Sicherungssignal elektrisch verbindet, wenn die Schalt-Kontrolleinheit (116) das Sicherungssignal an den Sicherungseingang des Sicherungs-Schalters ausgibt; wobei die Schalt-Kontrolleinheit ausgebildet ist, dieses Sicherungssignal zu erzeugen und auszugeben, **dadurch gekennzeichnet, dass**
der spannungsfeste Schalter (101) einen zweiten MOS-Sicherungstransistor (113) aufweist, welcher mit dem ersten MOS-Sicherungstransistor (114) derart in Reihe geschaltet ist, dass der Source-Anschluss des zweiten MOS-Sicherungstransistors (113) mit dem Source-Anschluss des ersten MOS-Sicherungstransistors (114) verbunden ist, so dass der Drain-Anschluss des zweiten MOS-Sicherungstransistors (113) mit dem Source-Anschluss wenigstens des ersten MOS-Schalttransistors (111) und der Drain-Anschluss des ersten MOS-Sicherungstransistors (114) mit dem Gate-Anschluss wenigstens des ersten MOS-Schalttransistors (111) verbunden ist und der Gate-Anschluss des ersten MOS-Sicherungstransistors (114) mit dem Gate-Anschluss des zweiten MOS-Sicherungstransistors (113) verbunden ist.

2. Spannungsfester Schalter nach Anspruch 1, **dadurch gekennzeichnet, dass** die Schalt-Kontrolleinheit ausgebildet ist, das Sicherungssignal zu erzeugen, wenn der erste MOS-Schalttransistor (111) sperrt.

3. Spannungsfester Schalter nach Anspruch 1, **dadurch gekennzeichnet, dass** der Sicherungsschalter (114) ein MOS-Sicherungstransistor ist, dessen Schalteingang durch einen Gate-Anschluss gebildet ist,
und der Drain-Anschluss des ersten MOS-Sicherungstransistors (114) mit dem Gate-Anschluss des ersten MOS-Schalttransistors (111) verbunden ist und der Source-Anschluss des ersten MOS-Sicherungstransistors (114) mit dem Source-Anschluss des ersten MOS-Schalttransistors (111) verbunden ist,
und die Schalt-Kontrolleinheit (116) ausgebildet ist, im Sperrfall des ersten MOS-Schalttransistors (111) den Gate-Anschluss des ersten MOS-Sicherungstransistors (114) derart mit einem Durchschaltpotential zu beaufschlagen, dass der erste MOS-Sicherungstransistor (114) durchsteuert und im Falle der Durchsteuerung des ersten MOS-Schalttransistors (111) den Gate-Anschluss des ersten MOS-Sicherungstransistors (114) derart mit einem Sperrpotential zu beaufschlagen, dass der erste MOS-Sicherungstransistor sperrt.

4. Spannungsfester Schalter (101) nach Anspruch 1, **dadurch gekennzeichnet, dass :**
die Schalt-Kontrolleinheit (116) zusätzlich einen Steuerausgang (126) aufweist, der mit dem Gateanschluss des ersten MOS-Schalttransistors (111) verbunden ist,
wobei die Schalt-Kontrolleinheit (116) ausgebildet ist, zur Durchsteuerung oder zur Sperrung des ersten MOS-Schalttransistors (111) zwischen dem Source-Anschluss und dem Drain-Anschluss, den Gate-Anschluss mit einem Steuerpotential zu beaufschlagen.

5. Spannungsfester Schalter (101) nach Anspruch 4, **dadurch gekennzeichnet, dass**
die Schalt-Kontrolleinheit (116) ausgebildet ist, das Steuerpotential aus einer Anzahl vorbestimmter Steuerpotentiale in Abhängigkeit von einem vorgegebenen Schaltpotential oder einem an dem ersten Schaltkontakt (120) anliegenden oder anzulegenden ersten Potential und/oder einem an dem zweiten Schaltkontakt (122) anliegenden oder anzulegenden zweiten Potential in vorgegebener Weise auszuwählen.

6. Spannungsfester Schalter nach einem der vorhergehenden Ansprüche, **dadurch gekennzeichnet, dass**
der spannungsfeste Schalter (101) einen zweiten MOS-Schalttransistor (112) aufweist, welcher mit dem ersten MOS-Schalttransistor (111) derart in Reihe geschaltet ist, dass der Source-Anschluss des zweiten MOS-Schalttransistors (112) mit dem Source-Anschluss des ersten MOS-Schalttransistors (111) verbunden ist, so dass der Drain-Anschluss des ersten MOS-Schalttransistors (111) mit dem ersten Schaltkontakt (120) und der Drain-Anschluss des zweiten MOS-Schalttransistors (112) mit dem zweiten Schaltkontakt (122) verbunden ist.

7. Spannungsfester Schalter (101) nach Anspruch 6, **dadurch gekennzeichnet, dass** der Gate-Anschluss des zweiten Schalttransistors (112) separat mit der Schalt-Kontrolleinheit (116) verbunden ist und die Schalt-Kontrolleinheit (116) ausgebildet ist, in Abhängigkeit von einem an dem ersten Schaltkontakt anzulegenden oder anliegenden Potential und/oder einem an dem zweiten Schaltkontakt anzulegenden oder anliegenden Potential die Gate-Anschlüsse der Schalttransistoren (111,112) jeweils unabhängig voneinander mit einem für den jeweiligen Schalttransistor vorbestimmten Steuerpotential zu beaufschlagen.

8. Spannungsfester Schalter (101) nach Anspruch 7, **dadurch gekennzeichnet, dass** die Schalt-Kontrolleinheit einen Mittenpotentialanschluss aufweist, welcher mit den Source-Anschlüssen der Schalttransistoren (111, 112) verbunden ist, wobei die Schalt-Kontrolleinheit ausgebildet ist, beim Sperren der Schalttransistoren den Mittenpotentialanschluss derart mit einem Mittenpotential zu beaufschlagen, dass jeweils eine vorbestimmte Potentialdifferenz zwischen dem Mittenpotentialanschluss und den Gate-Anschlüssen der Schalttransistoren nicht überschritten wird.

9. Spannungsfester Schalter nach Anspruch 6, **dadurch gekennzeichnet, dass** der Gate-Anschluss des ersten Schalttransistors (111) mit dem Gate-Anschluss des zweiten MOS-Schalttransistors (112) verbunden ist.

10. Spannungsfester Schalter nach einem der vorhergehenden Ansprüche, **dadurch gekennzeichnet, dass** jeder MOS-Schalttransistor (111, 112) einen Bulkanschluss aufweist, wobei der Bulk-Anschluss mit dem Source-Anschluss verbunden ist.

11. Spannungsfester Schalter nach einem der Ansprüche 2 bis 9, **dadurch gekennzeichnet, dass** jeder MOS-Sicherungstransistor (113, 114) einen Bulk-Anschluss aufweist, wobei der Bulk-Anschluss mit dem Source-Anschluss verbunden ist.

12. Spannungsfester Schalter nach einem der vorhergehenden Ansprüche, **dadurch gekennzeichnet, dass** die MOS-Schalttransistoren (111, 112) und die MOS-Sicherungstransistoren (113, 114) als unsymmetrische MOS-Transistoren ausgebildet sind.

13. Spannungsfester Schalter nach einem der vorhergehenden Ansprüche, **dadurch gekennzeichnet, dass** die MOS-Schalttransistoren (111, 112) und die MOS,-Sicherungstransistoren (113, 114) als Insulated Gate Bipolar Transistoren ausgebildet sind.

14. Spannungsfester Schalter nach einem der vorhergehenden Ansprüche, **dadurch gekennzeichnet, dass** der spannungsfeste Schalter monolithisch integriert ist.

15. Herzschrittmacher oder Defibrillator mit einem spannungsfesten Schalter nach einem der vorhergehenden Ansprüche.

16. Stimulationseinheit (401) zur Stimulation eines Herzens, welche einen Kondensator (406) zur Speicherung elektrischer Ladung für ein Stimulationssignal und einen spannungsfesten Schalter (101, 404) nach Anspruch 1 bis 14 aufweist, wobei der spannungsfeste Schalter (101, 404) derart mit dem Kondensator (406) wirkverbunden ist, dass eine Entladung des Kondensators (406) über den spannungsfesten Schalter (101, 404) erfolgen kann.

17. Stimulationseinheit (401) nach Anspruch 16 mit einer Steuereinheit (402), wobei die Steuereinheit (402) mit dem spannungsfesten Schalter (101, 404) verbunden und ausgebildet ist, Stimulationssignale zur Stimulation eines Herzens mit dem spannungsfesten Schalter (101, 404) zu schalten.

## Claims

1. A voltage-proof switch (101), comprising
a first switch contact (120) and a second switch contact (122),
a first MOS switching transistor (111) having a source terminal, a drain terminal and a gate terminal, the source terminal of the first MOS switching transistor (111) being connected to the second switch contact (122), and the drain terminal of the first MOS switching transistor (111) being connected to the first switch contact (120), and the voltage-proof switch being designed for switching a potential that is or will be applied to the first or second switch contact, wherein the voltage-proof switch (101) has a switch control unit (116) with a control input (124) and a fuse output (129), which is connected to a switch input of a fuse switch (114), this fuse switch (114) being arranged and designed so that it electrically connects the gate terminal of the first MOS switching transistor (111) to the source terminal of the first MOS switching transistor (111) as a function of a protection signal when the switch control unit (116) outputs the protection signal to the fuse input of the fuse switch, the switch control unit being designed to generate and output this protection signal, **characterized in that**
the voltage-proof switch (101) has a second MOS fuse transistor (113), which is connected in series with the first MOS fuse transistor (114) such that the source terminal of the second MOS fuse transistor (113) is connected to the source terminal of the first MOS fuse transistor (114) so that the drain terminal of the second MOS fuse transistor (113) is connected to the source terminal of at least the first MOS switching transistor (111) and the drain terminal of the first MOS fuse transistor (114) is connected to the gate terminal of at least the first MOS switching transistor (111), and the gate terminal of the first MOS fuse transistor (114) is connected to the gate terminal of the second MOS fuse transistor (113).

2. The voltage-proof switch according to claim 1, **characterized in that** the switch control unit is designed to generate the protection signal when the first MOS switching transistor (111) is nonconducting.

3. The voltage-proof switch according to claim 1, **characterized in that** the fuse switch (114) is a MOS fuse transistor whose switch input is formed by a gate terminal,
and the drain terminal of the first MOS fuse transistor (114) is connected to the gate terminal of the first MOS switching transistor (111), and the source terminal of the first MOS fuse transistor (114) is connected to the source terminal of the first MOS switching transistor (111),
and the switch control unit (116) is designed to apply a switching-through potential to the gate terminal of the first MOS fuse transistor (114) in the nonconducting state of the first MOS switching transistor (111), such that the first MOS fuse transistor (114) is turned on, and in the case of the first MOS switching transistor (111) being turned on, a blocking-state potential is applied to the gate terminal of the first MOS fuse transistor (114), such that the first MOS fuse transistor is nonconducting.

4. The voltage-proof switch (101) according to claim 1, **characterized in that**
the switch control unit (116) additionally has a control output (126) connected to the gate terminal of the first MOS switching transistor (111),
the switch control unit (116) being designed to apply a control potential to the gate terminal for turning on or blocking of the first MOS switching transistor (111) between the source terminal and the drain terminal.

5. The voltage-proof switch (101) according to claim 4, **characterized in that**
the switch control unit (116) is designed to select the control potential from a number of predetermined control potentials as a function of a predetermined switch potential or a first potential that is or will be applied to the first switch contact (120) and/or a second potential that is or will be applied to the second switch contact (122) in a predetermined manner.

6. The voltage-proof switch according to any one of the preceding claims, **characterized in that**
the voltage-proof switch (101) has a second MOS switching transistor (112) connected in series with the first MOS switching transistor (111), such that the source terminal of the second MOS switching transistor (112) is connected to the source terminal of the first MOS switching transistor (111), such that the drain terminal of the first MOS switching transistor (111) is connected to the first switch contact (120), and the drain terminal of the second MOS switching transistor (112) is connected to the second switch contact (122).

7. The voltage-proof switch (101) according to claim 6, **characterized in that** the gate terminal of the second switching transistor (112) is connected separately to the switch control unit (116), and the switch control unit (116) is designed to apply a control potential that is predetermined for the respective switching transistor to the gate terminals of the switching transistors (111, 112), independently of one another, as a function of a potential that is or will be applied to the first switch contact and/or a potential that is or will be applied to the second switch contact.

8. The voltage-proof switch (101) according to claim 7, **characterized in that** the switch control unit has a mid-potential terminal connected to the source terminals of the switching transistors (111, 112), such that the switch control unit is designed to apply a mid-potential to the mid-potential terminal in blocking of the switching transistors, such that a predetermined potential difference between the mid-potential terminal and the gate terminals of the switching transistors is not exceeded.

9. The voltage-proof switch according to claim 6, **characterized in that** the gate terminal of the first switching transistor (111) is connected to the gate terminal of the second MOS switching transistor (112).

10. The voltage-proof switch according to any one of the preceding claims, **characterized in that** each MOS switching transistor (111, 112) has a bulk terminal, the bulk terminal being connected to the source terminal.

11. The voltage-proof switch according to any one of claims 2 to 9, **characterized in that** each MOS fuse transistor (113, 114) has a bulk terminal, the bulk terminal being connected to the source terminal.

12. The voltage-proof switch according to any one of the preceding claims, **characterized in that** the MOS switching transistors (111, 112) and the MOS fuse transistors (113, 114) are designed as asymmetric MOS transistors.

13. The voltage-proof switch according to any one of the preceding claims, **characterized in that** the MOS switching transistors (111, 112) and the MOS fuse transistors (113, 114) are designed as insulated gate bipolar transistors.

14. The voltage-proof switch according to any one of the preceding claims, **characterized in that** the voltage-proof switch is monolithically integrated.

15. Cardiac pacemaker or defibrillator having a voltage-proof according to any one of the preceding claims.

16. Stimulation unit (401) for stimulation of the heart, having a capacity (406) for storing electric charge for a stimulation signal and a voltage-proof switch (101, 404) according to claims 1 to 14, wherein the voltage-proof switch (101, 404) is functionally connected to the capacitor (406) such that discharging of the capacitor (406) may occur across the voltage-proof switch (101, 404).

17. The stimulation unit (401) according to claim 16, comprising a controller unit (402), the controller unit (402) being connected to the voltage-proof switch (101, 404) and being designed to switch stimulation signals for stimulation of the heart to a voltage-proof switch (101, 404).

## Revendications

1. Interrupteur à tension fixe (101) comportant
un premier contact de commutation (120) et un deuxième contact de commutation (122),
un premier transistor de commutation MOS (111) doté d'un raccordement source, d'un raccordement drain et d'un raccordement portillon, le raccordement source du premier transistor de commutation MOS (111) étant relié au deuxième contact de commutation (122) et le raccordement drain du premier transistor de commutation MOS (111) au premier contact de commutation (120), l'interrupteur à tension fixe étant conçu pour commuter un potentiel à appliquer ou appliqué au premier ou au deuxième contact de commutation,
l'interrupteur à tension fixe (101) présentant une unité de contrôle de commutation (116) dotée d'une entrée de commande (124) et d'une sortie de coupe-circuit (129) qui est reliée à une entrée de commutation d'un interrupteur coupe-circuit (114), cet interrupteur coupe-circuit (114) étant disposé et réalisé de manière à relier électriquement le raccordement portillon du premier transistor de commutation MOS (111) au raccordement source du premier transistor de commutation MOS (111) en fonction d'un signal de coupe-circuit lorsque l'unité de contrôle de commutation (116) envoie le signal de coupe-circuit à l'entrée de coupe-circuit de l'interrupteur coupe-circuit, l'unité de contrôle de commutation étant conçue pour générer et émettre ce signal de coupe-circuit,
**caractérisé en ce que**
l'interrupteur à tension fixe (101) présente un deuxième transistor de coupe-circuit MOS (113) qui est commuté en série avec le premier transistor de coupe-circuit MOS (114) de manière à relier le raccordement source du deuxième transistor de coupe-circuit MOS (113) au raccordement source du premier transistor de coupe-circuit MOS (114), de sorte que le raccordement drain du deuxième transistor de coupe-circuit MOS (113) est relié au raccordement source au moins du premier transistor de commutation MOS (111) et que le raccordement drain du premier transistor de coupe-circuit MOS (114) est relié au raccordement portillon au moins du premier transistor de commutation MOS (111) et que le raccordement portillon du premier transistor de coupe-circuit MOS (114) est relié au raccordement portillon du deuxième transistor de coupe-circuit MOS (113).

2. Interrupteur à tension fixe selon la revendication 1, **caractérisé en ce que** l'unité de contrôle de commutation est conçue pour générer un signal de coupe-circuit lorsque le premier transistor de commutation MOS (111) fait barrage.

3. Interrupteur à tension fixe selon la revendication 1, **caractérisé en ce que** l'interrupteur de coupe-circuit (114) est un transistor de coupe-circuit MOS dont l'entrée de commutation est constituée par un raccordement portillon
et que le raccordement drain du premier transistor de coupe-circuit MOS (114) est relié au raccordement portillon du premier transistor de commutation MOS (111) et que le raccordement source du premier transistor de coupe-circuit MOS (114) est relié au raccordement source du premier transistor de commutation MOS (111)
et que l'unité de commande de commutation (116) est conçue pour, en cas de barrage du premier transistor de commutation MOS (111), solliciter le raccordement portillon du premier transistor de coupe-circuit MOS (114) avec un potentiel de transit de commutation de manière à ce que le premier transistor de coupe-circuit MOS (114) commute et, dans le cas de commutation du premier transistor de commutation MOS (111), à solliciter le raccordement portillon du premier transistor de coupe-circuit MOS (114) avec un potentiel de barrage pour que le premier transistor de coupe-circuit MOS fasse barrage.

4. Interrupteur à tension fixe (101) selon la revendication 1, **caractérisé en ce que :**
l'unité de commande de commutation (116) présente en outre une sortie de commande (126) qui est reliée au raccordement portillon du premier transistor de commutation MOS (111),
l'unité de commande de commutation (116) étant conçue pour commander ou pour barrer le premier transistor de commutation MOS (111) entre le raccordement source et le raccordement drain et pour solliciter le raccordement portillon avec un potentiel de commande.

5. Interrupteur à tension fixe (101) selon la revendication 4, **caractérisé en ce que** :
l'unité de commande de commutation (116) est conçue pour sélectionner de manière prédéfinie le potentiel de commande en fonction d'un potentiel de commande prédéfini ou d'un premier potentiel appliqué ou à appliquer au premier contact de commutation (120) et/ou d'un deuxième potentiel appliqué ou à appliquer au deuxième contact de commutation (122) d'une manière prédéfinie.

6. Interrupteur à tension fixe selon une des revendications précédentes, **caractérisé en ce que**
l'interrupteur à tension fixe (101) présente un deuxième transistor de commutation MOS (112) qui est commuté en série avec le premier transistor de commutation MOS (111) de manière à ce que le raccordement source du deuxième transistor de commutation MOS (112) soit relié au raccordement source du premier transistor de commutation MOS (111), de sorte que le raccordement drain du premier transistor de commutation MOS (111) est relié au premier contact de commutation (120) et le raccordement drain du deuxième transistor de commutation MOS (112) est relié au deuxième contact de commutation (122).

7. Interrupteur à tension fixe (101) selon la revendication 6, **caractérisé en ce que** le raccordement portillon du deuxième transistor de commutation MOS (112) est relié séparément à l'unité de contrôle de commutation (116) et que l'unité de contrôle de commutation (116) est conçue pour, en fonction d'un potentiel appliqué ou à appliquer au premier contact de commutation et/ou d'un potentiel appliqué ou à appliquer au deuxième contact de commutation, solliciter respectivement indépendamment les uns des autres les raccordements à portillon avec un potentiel de commande prédéfini pour le transistor de commutation (111, 112) respectif.

8. Interrupteur à tension fixe (101) selon la revendication 7, **caractérisé en ce que** l'unité de contrôle de commutation présente un raccordement à potentiel central qui est relié aux raccordements source des transistors de commutation (111, 112), l'unité de contrôle de commutation étant conçue pour, en cas de barrage des transistors de commutation, solliciter le raccordement à potentiel central avec un potentiel central de manière à ce que respectivement une différence de potentiel prédéfinie entre le raccordement à potentiel central et les raccordements portillon des transistors de commutation ne soit pas dépassée.

9. Interrupteur à tension fixe selon la revendication 6, **caractérisé en ce que** le raccordement portillon du premier transistor de commutation MOS (111) est relié au raccordement portillon du deuxième transistor de commutation MOS (112).

10. Interrupteur à tension fixe selon une des revendications précédentes, **caractérisé en ce que** chaque transistor de commutation MOS (111, 112) présente un raccordement bulk, le raccordement bulk étant relié au raccordement source.

11. Interrupteur à tension fixe selon une des revendications 2 à 9, **caractérisé en ce que** chaque transistor de coupe-circuit MOS (113, 114) présente un raccordement bulk, le raccordement bulk étant relié au raccordement source.

12. Interrupteur à tension fixe selon une des revendications précédentes, **caractérisé en ce que** les transistors de commutation MOS (111, 112) et les transistors de coupe-circuit MOS (113, 114) se présentent sous forme de transistors MOS asymétriques.

13. Interrupteur à tension fixe selon une des revendications précédentes, **caractérisé en ce que** les transistors de commutation MOS (111, 112) et les transistors de commutation MOS (113, 114) se présentent sous forme de transistors bipolaires à portillons isolés.

14. Interrupteur à tension fixe selon une des revendications précédentes, **caractérisé en ce que** l'interrupteur à tension fixe est intégré de manière monolithique.

15. Stimulateur cardiaque ou défibrillateur comportant un interrupteur à tension fixe selon une des revendications précédentes.

16. Unité de stimulation (401) pour la stimulation d'un coeur, présentant un condensateur (406) pour l'accumulation d'une charge électrique pour un signal de stimulation et un interrupteur à tension fixe (101, 404) selon les revendications 1 à 14, l'interrupteur à tension fixe (101, 404) étant en relation interactive avec le condensateur (406) de manière à ce qu'une décharge du condensateur (406) puisse avoir lieu via l'interrupteur à tension fixe (101, 404).

17. Unité de stimulation (401) selon la revendication 16, comportant une unité de commande (402), l'unité de commande (402) étant reliée à l'interrupteur à tension fixe (101, 404) et conçue pour commuter des signaux de stimulation pour la stimulation d'un coeur à l'aide de l'interrupteur à tension fixe (101, 404).
